# EUROPÄISCHE PATENTANMELDUNG

(11) **EP 1 378 259 A1**
(43) Veröffentlichungstag der Anmeldung: **07.01.2004**
(21) Anmeldenummer: 03090197.9
(22) Anmeldetag: 04.07.2003
(51) Int. Cl.: A61M 3/02, F04B 49/10

(54) **Fluidpumpe für medizinische Anwendungen und Messkammer dafür**

(30) Priorität: 05.07.2002 DE 10231461
(71) Anmelder: Korejwo, Richard, 13465 Berlin (DE)
(72) Erfinder: Korejwo, Richard, 13465 Berlin (DE)
(74) Vertreter: Pfenning, Meinig & Partner

(57) **Zusammenfassung**

Fluidpumpe für medizinische, insbesondere endoskopische Anwendungen, mit einem über einen Antrieb geführten Förderkanal, einer eine Messkammer aufweisenden Vorrichtung zur Messung des Drucks des im Förderkanal geförderten Fluids und Steuermitteln zur automatischen Steuerung des Pumpenverhaltens, insbesondere des Förderverhaltens, in Abhängigkeit vom gemessenen Druck und in Abhängigkeit von einem Codewert, welcher in Codewert-Träger-Mitteln enthalten ist, die an oder in der Meßkammer vorgesehenen sind, wobei der Codewert während des und/oder nach dem Festlegen der Messkammer am Pumpengehäuse durch Auslesemittel der Fluidpumpe erfaßt wird.

## Beschreibung

Die Erfindung betrifft eine Fluidpumpe nach dem Oberbegriff des Patentanspruchs 1 sowie eine Messkammer nach Anspruch 7.

Aus der Patentschrift DE 195 25 926 C1 ist ein Peristaltik-Pumpensystem bekannt, bei dem in die Schlauchleitung des Pumpensystems nach der Pumpe eine Meßvorrichtung eingesetzt ist. Diese der Pumpe nachgeschaltete Meßvorrichtung ermittelt durch Druckmessung das Fördervolumen der Pumpe an Fluid. Die Meßvorrichtung ist dabei in einfacher Weise blockmäßig aufgebaut, so daß sie aus einer Halterung entfernt werden kann, um das Meßgehäuse unkompliziert desinfizieren zu können. Die Übertragung des Drucks an Fluid aus dem Inneren des Meßgehäuses nach außen erfolgt dabei über Öffnungen, welche mit einer Membran dicht abgedeckt sind.

Die Messgehäuse können dabei als zu desinfizierende Mehrweg-Messkammern, aber auch als steril verpackte Einweg-Produkte realisiert sein. Durch die Entnehmbarkeit der Messkammer, bzw. der Ausgestaltung der Messkammer als separates, austauschbares Zubehörteil entsteht jedoch der besonders für den medizinischen Bereich kritische und schwerwiegende Nachteil, dass versehentlich eine Messkammer, die eigentlich für ein bestimmtes, erstes Pumpensystem bestimmt und geeignet ist, in ein anderes Pumpensystem eingesetzt wird, wodurch die bestimmungsgemäße Funktion des letztgenannten Pumpensystems in Frage gestellt wird.

Dies ist beispielsweise insbesondere dann der Fall, wenn Pumpensysteme mit den jeweiligen Messkammern als Zubehör von verschiedenen Herstellern vorhanden sind, wobei die Messkammern eines ersten Herstellers in die Pumpensysteme eines anderen Herstellers einsetzbar sind (hier insbesondere, wenn die äußeren Abmessungen der Messkammern der verschiedenen Hersteller untereinander identisch sind), jedoch für die Druckmessung bedeutsame Eigenschaften der Messkammern zwischen den Herstellern differieren. Dadurch kann es zu Fehlfunktionen der Pumpensysteme kommen.

Es ist somit Aufgabe der vorliegenden Erfindung, eine Fluidpumpe mit einer Druckmesskammer und eine Druckmesskammer hierfür anzugeben, welche besonders einfach und robust in der praktischen klinischen Handhabung ist, bei der die Messkammern besonders kostengünstig in industrieller Massenfertigung herzustellen sind und wobei gleichzeitig das versehentliche Betreiben einer Messkammer in einer für diese Messkammer ungeeigneten Pumpe verhindert wird.

Diese Aufgaben werden durch eine Fluidpumpe nach Patentanspruch 1 und eine Messkammer nach Patentanspruch 16 erfindungsgemäß gelöst.

Die Erfindung nach Patentanspruch 1 weist dabei die folgenden vorteilhaften Merkmale auf:

Dadurch, dass an der Messkammer der erfindungsgemäßen Fluidpumpe Codewert-Träger-Mittel vorgesehen sind, wird der besondere Vorteil erreicht, dass Codewerte in diese Codewert-Träger-Mittel eingegeben sein können und somit die Messkammer individualisiert werden kann. Solche Codewerte können alphanumerische Werte sein, oder andere Werte, welche Informationen über Bauart und/oder Leistungsmerkmale der Messkammer oder deren Hersteller tragen. Dadurch, dass die Codewert-Träger-Mittel während und/oder nach dem Festlegen am Pumpengehäuse der Fluidpumpe - beispielsweise durch Einsetzen in eine Haltevorrichtung der Fluidpumpe - von den Auslesemitteln der Fluidpumpe erfaßt werden können, wird ein im praktischen klinischen Bereich besonders unpraktischer zusätzlicher Arbeitsschritt von vornherein vermieden, beispielsweise das Herstellen einer elektrischen Kabelverbindung zwischen den Codewert-Träger-Mitteln und der Fluidpumpe oder etwa das manuelle Auslesen eines auf dem Gehäuse der Messkammer aufgebrachten Scancodes durch einen mit der Fluidpumpe verbundenen Handscanner.

Dadurch, dass Auslesemittel vorgesehen sind, welche den Codewert aus den Codewert-Träger-Mitteln auslesen können, kann die Fluidpumpe ihren Betrieb je nach dem in die Codewert-Träger-Mittel der Messkammer eingegebenen Codewert ihr Funktionsverhalten auf die eingesetzte Messkammer abstimmen. Dieses Abstimmen kann etwa in einer Veränderung des Förderverhaltens der Pumpe bestehen oder auch darin, dass die Pumpe ihren Förderbetrieb verweigert.

Die Festlegbarkeit der Druckmesskammer am Pumpengehäuse kann etwa dadurch erreicht werden, dass das Gehäuse der Messkammer und die Fluidpumpe so ausgestattet ist, dass die Messkammer in eine Haltevorrichtung an der Fluidpumpe einsetzbar ist. So ist sie besonders einfach und ohne zusätzlichen Befestigungsaufwand in eine Schlauchleitung hineinzuschalten und ihr wieder zu entnehmen, ohne dass dabei die Länge der Schlauchleitung nennenswert vergrößert wird. Eine solche Gehäusegestaltung kann etwa dadurch erreicht werden, dass das kastenförmige Gehäuse eine glatte und robuste Oberfläche aufweist, die kratz- und bruchsicher ist, und bei der auf zerbrechliche Außenteile verzichtet wird.

Vorteilhafte Weiterbildungen der Erfindung nach Anspruch 1 sind nach den auf diesen rückbezogenen Unteransprüchen möglich und werden im Folgenden erläutert.

Die Anpassung des Pumpenverhaltens wird durch die Funktionen der Steuermittel der Fluidpumpe bewirkt, welche das Verhalten der Fluidpumpe abhängig vom durch die Auslesemittel erfassten und in den Codewert-Träger-Mitteln der Messkammer vorgehaltenen Codewert steuern. Auf diese Weise wird es ermöglicht, durch die Verwendung verschiedener Messkammern mit zumindest äußerlich gleichen geometrischen Abmessungen unter Verwendung verschiedener Codewerte das Betriebsverhalten der Pumpe zu steuern: etwa dadurch, dass eine erste Messkammer mit einem ersten Codewert die Fördereigenschaften der Pumpe für Eingriffe im Bereich des zentralen Nervensystems auslöst und alternativ eine zweite Messkammer mit einem zweiten Codewert in derselben Fluidpumpe die Fördereigenschaften für endoskopische Eingriffe im Knie auslöst.

Auf diese Weise kann auch effektiv verhindert werden, dass eine irrtümliche Verwendung einer nicht zur Pumpe gehörenden Messkammer dazu führt, dass beispielsweise der Druck des geförderten Fluids und damit die Fördermenge falsch bestimmt wird oder eine Messkammer im Zusammenhang mit der Fluidpumpe verwendet wird, welche nicht den durch den Pumpenhersteller geforderten und spezifizierten Eigenschaften genügt.

Eine besonders einfache Realisierung des Zusammenspiels aus Codewert-Träger-Mitteln und Auslesemitteln ist es, die Codewert-Träger-Mittel als eine oder mehrere, beispielsweise stiftförmige, Ausformungen des Messkammergehäuses zu realisieren, die durch eine entsprechend komplementäre Ausformung des Pumpengehäuses ein vollständiges Festlegen der Messkammer am Pumpengehäuse nur dann erlauben, wenn das durch die Stifte gebildete (dem Codewert entsprechende) Muster dem durch die Ausformung des Pumpengehäuses vorgegebenen entspricht. Die Fluidpumpe kann dann so realisiert sein, daß sie ihren Förderbetrieb nur dann aufnimmt, wenn eine Messkammer vollständig an ihr festgelegt ist.

Andere Realisierungsformen der Steuermittel, insbesondere wenn sie durch dazu geeignete Funktionen elektronischer Steuermittel verwirklicht sind, beruhen auf dem Prinzip, daß aus den Codewert-Träger-Mitteln ein darin enthaltener Codewert durch die Auslesemittel erfaßt wird und vermittels einer geeigneten Steuerungslogik überprüft wird, ob dieser Codewert (bzw. diese Menge an Codewerten) mit dem oder den von der Steuerungslogik der Fluidpumpe erwarteten Codewert(en) übereinstimmt und/oder darin enthalten ist und daraufhin durch einfaches Einsetzen einer in geeigneter Weise codierten Messkammer das Förderverhalten der Fluidpumpe dem gewünschten medizinischen Einsatz gemäß angepaßt wird, oder ein Blockieren der Pumpe ausgelöst wird, so daß der sichere Betrieb der Pumpe gewährleistet werden kann.

Eine besonders vorteilhafte Weise zur Realisierung ist es, wenn die Codewert-Träger-Mittel Mittel zur optischen und/oder elektrischen und/oder magnetischen und/oder mechanischen Codewertspeicherung enthalten, sowie ebenfalls Mittel zur Übertragung eines Codewerts auf einer der vorgenannten Arten und Weisen, soweit diese von den Auslesemitteln der Fluidpumpe nicht unmittelbar aus den Codewert-Träger-Mitteln ausgelesen werden können.

Dabei müssen die Auslesemittel nicht zwingend das gleiche Realisierungsprinzip wie die Codewert-Träger-Mittel verkörpern, jedoch an diese angepasst sein. So ist etwa die mechanische Codewertspeicherung durch Erhebungen am Gehäuse denkbar, welche optisch erfasst wird, oder alternativ eine mechanische Abtastung einer magnetischen Speicherung.

Besonders vorteilhaft ist es, wenn der in den Codewert-Träger-Mitteln enthaltene Codewert - ebenso sind mehrere Codewerte gleichzeitig denkbar - Informationen hinsichtlich der technischen Eigenschaften der Messkammer (wie Durchströmquerschnitt des geförderten Fluids oder Querschnitt der Messdüse) und/oder hinsichtlich des Herstellers (Messkammerhersteller oder Fluidpumpenhersteller) und/oder hinsichtlich der beabsichtigten Funktionsweise der Pumpe (gewünschtes Fördervolumen, erlaubte Förder-Toleranz bei verschiedenen medizinischen Anwendungsgebieten) repräsentiert.

Eine praktische und besonders kostengünstige Variante der Realisierung der Codierungsmittel ist etwa, am Gehäuse der Messkammer mechanisch abtastbare Erhebungen und/oder Vertiefungen und/oder Ausfassungen vorzusehen. Hierdurch wird eine aufwandsarme und robuste Lösung für die Codewert-Träger-Mittel gewählt, welche in ihrer Funktion durch Feuchtigkeit und/oder Chemikalien, Strahlungen und Temperatur nicht beeinträchtigt wird. Werden diese Codewert-Träger-Mittel an einer Seite des Messkammer-Gehäuses angeordnet, so dass sie in direktem Kontakt mit den Auslesemitteln der Fluidpumpe stehen, so kann die Abtastung der Code-Stifte durch elektrische Druckkontakte, etwa wie bei einer Folientastatur, erfolgen. Besonders vorteilhaft ist eine Anordnung von Codewert-Träger-Mitteln und Auslesemitteln, derart, dass diese in Wirkverbindung miteinander unmittelbar durch das Festlegen der Messkammer am oder im Gehäuse der Fluidpumpe und ohne weiteres Zutun zu stellen sind.

Es sind jedoch auch andere Realisierungen denkbar, etwa die Verwendung eines Barcodes als Codewert-Träger-Mittel und einen Barcodeleser als Auslesemittel der Fluidpumpe sowie eine Repräsentierung des Codewerts in den Codewert-Träger-Mitteln durch Färbungen. Bei diesen beiden Codierungsarten ist von Vorteil, daß der Barcode bzw. die Färbungen als Realisierung der Codewert-Träger-Mittel eine sehr kostengünstige Realisierungsform auf der Seite der Messkammer darstellen, was deswegen von Bedeutung ist, weil die Messkammern im Vergleich zu den Fluidpumpen in sehr großen Stückzahlen hergestellt werden.

Eine etwas aufwendigere Realisierungsform sieht vor, für die Codewert-Träger-Mittel eine integrierte Schaltung vorzusehen, in der der mindestens eine Codewert gespeichert ist. Hierbei ist von Vorteil, daß die Menge an Codewerten bzw. an im Codewert enthaltenen Informationen sehr viel umfangreicher sein kann. In diesem Zusammenhang ist eine besonders vorteilhafte Realisierungsform der Übertragung des Codeswertes aus den Codewert-Träger-Mitteln an die Auslesemittel durch elektromagnetische Signale.

Besonders hinsichtlich des praktischen klinischen Einsatzes der Messkammern ist es besonders vorteilhaft, das Gehäuse der Messkammer mit Mitteln zu versehen zur eindeutigen Kennzeichnung der Orientierung des Gehäuses zum Fluidpumpengehäuse. Hierdurch wird es auf einfache Weise verhindert, dass beim Festlegen der Messkammer an oder in der Pumpe, z.B. in eine hierfür vorgesehene Haltevorrichtung der Pumpe und durch eine translatorische Bewegung der Messkammer in Längsrichtung des Messkammergehäuses, verkehrt herum eingesetzt wird, insbesondere die Kammer entgegen der vorgesehenen Strömungsrichtung in den Schlauchkreislauf geschaltet wird. Durch das Vorsehen solcher Mittel entfällt eine besondere Vorsicht des Verwenders beim Einsetzen. Sie können z.B. durch besondere Formgebung des Gehäuses im Zusammenhang mit einer komplementären Formgebung an der Fluidpumpe sein, wie etwa die Verwendung unsymmetrischer Geometrieeigenschaften der Gehäusegeometrien,

Eine weitere vorteilhafte Weiterbildung der Messkammer sieht vor, eine Membran an der Messkammer vorzusehen, auf die der Druck aus dem Inneren der Messkammer mittels hierfür vorgesehener Öffnungen des Messkammergehäuses weitergegeben werden kann, wobei die Öffnungen durch die Membran dicht abgedeckt werden, so dass durch die Dichtigkeit der Abdeckungen der Öffnungen mit der Membran das geförderte Fluid auch im Bereich der Messkammer in einem geschlossenen System verbleibt.

In Kombination mit der Membran und besonders in Hinsicht auf den praktischen Gebrauch ist es vorteilhaft, die Messkammer zusätzlich mit einem die Membran vollständig abdeckenden Membranschutz auszustatten, wodurch die Membran der Messkammer im praktischen Gebrauch, wie Transport oder Desinfektion, sicher geschützt und ein unbeabsichtigtes Einreißen oder Ausbeulen der Membran verhindert wird. Besonders vorteilhaft ist es hierbei, das Öffnen des Membranschutzes durch ein seitliches Verschieben entlang des Messkammergehäuses zu realisieren.

Dies kann beispielsweise derart ausgeführt sein, dass der Membranschutz beim Festlegen an der Fluidpumpe automatisch veranlasst wird, die Membran freizugeben und damit der Fluidpumpe den Kontakt zur Membran, etwa für Drucksensoren der Fluidpumpe, ermöglicht wird. Es wird hierdurch insbesondere der Nachteil einer hüllen- oder haubenartigen Abdeckung vermieden, die von Hand aufwendig aufgesteckt oder entfernt werden muss und die insbesondere leicht verloren gehen kann. In einer analogen Art und Weise zum automatischen Öffnen des Membranschutzes beim Festlegen kann das Schließen des Membranschutzes beim Entnehmen der Messkammer aus oder von der Fluidpumpe realisiert werden.

Eine besonders praktikable Ausführungsform für die seitliche Verschiebbarkeit des als Platte ausgeführten Membranschutzes stellen Führungsschienen entlang des Messkammergehäuses dar, welche als Ausformungen des Messkammergehäuses realisiert sein können.

Durch das automatische Öffnen und Schließen des Membranschutzes der Messkammer bei deren Festlegen an oder in der Fluidpumpe entfällt ein unhandliches manuelles Beiseiteschieben oder Entfernen des Membranschutzes - bzw. das Wiederaufsetzen - , bei dem die Verwendung beider Hände des Verwenders erforderlich sein könnte.

Eine weitere vorteilhafte Ausführungsform der Messkammer sieht vor, an der Messkammer Rastmittel vorzusehen, die das Gehäuse nach dem Festlegen an einer Fluidpumpe durch Kraftschluss und/oder Formschluss halten. Vorzugsweise sind die Einrastmittel so ausgeführt, dass sie beim Festlegen ein deutlich vernehmbares Klicken erzeugen und das Festlegen und Entnehmen durch einfache und unmittelbare translatorische Kraftanwendung, insbesondere ohne ein Betätigen von zusätzlichen Entriegelungsvorrichtungen durchgeführt werden kann.

Weiterhin ist es vorteilhaft, ein Pumpsegment vorzusehen, welches eingangsseitig mit der Messkammer durch Klemmen fest verbunden ist. Dies hat den Vorteil, dass mit jedem Wechsel der Messkammer das Pumpsegment mit ausgewechselt wird. Dies ist besonders dann von Bedeutung, wenn der Antrieb der als Peristaltikpumpe ausgeführten Fluidpumpe über ein Rollenrad realisiert ist und deswegen an das als Schlauch ausgeführte Pumpsegment besonders hohe Anforderungen an die mechanischen Eigenschaften des Pumpsegmentes gestellt werden. Diese mechanischen Eigenschaften unterliegen jedoch einer besonders starken Alterung bzw. Abnutzung während des Gebrauches, so dass ein regelmäßiger Austausch sichergestellt werden muss. Dies wird in der vorliegenden Ausführungsform durch die feste Anbindung an die Messkammer realisiert.

Eine weitere vorteilhafte Ausführungsform sieht vor, in der Messkammer einen Strömungskanal vorzusehen und darin eine den Strömungsquerschnitt des durch den Strömungskanal durchströmenden Fluids verringernde Messdüse auszubilden, wobei vor und nach der Messdüse Öffnungen zum Äußeren der Messkammer angeordnet sind, welche die Druckmessung ermöglichen.

Die Merkmale der Erfindung nach Anspruch 16 weisen die folgenden vorteilhaften Wirkungen auf:

Dadurch, dass die Messkammer Codewert-Träger-Mittel enthält, in welchen mindestens ein Codewert enthalten ist und welche Mittel zur Codewertspeicherung und-Übermittlung enthalten, wird erreicht, dass die Messkammer durch den enthaltenen Codewert auf einfache Weise individualisiert werden kann und die Kompatibilität zwischen Messkammern und Fluidpumpen verschiedener Hersteller gezielt gesteuert werden kann, wie vorstehend bei den Ausführungen über die vorteilhaften Wirkungen der Fluidpumpe nach Anspruch 1 genauer beschrieben. Es kann Kompatibilität und Individualisierung auf diese Weise vorgenommen werden, ohne dass produktionstechnisch aufwendig Verschiedenartigkeiten des Gehäuses oder der mechanischen Passform hergestellt werden müssten. Hierdurch werden die Skaleneffekte industrieller Massenfertigung produktionskostenreduzierend ausgenutzt, da die Gehäuseherstellung für alle Messkammergehäuse des äußeren Abmessungen nach in gleicher Weise erfolgen kann und dadurch Rationalisierungseffekte in der Produktion eintreten.

Vorteilhafte Weiterbildung der Erfindung nach Anspruch 7 sind nach den auf diesen rückbezogenen Unteransprüchen und entsprechend der Weiterbildungen der Fluidpumpe, welche die Messkammern betreffen möglich.

Die Erfindung wird nachstehend an einem Ausführungsbeispiel erläutert. Es zeigt:
- Fig. 1: Eine keilförmige Meßkammer mit Membran, mit Membranschutz in geöffnetem und geschlossenem Zustand, sowie mit Codewert-Träger-Mitteln in Form von Code-Stiften,
- Fig. 2: eine halbtransparente Ansicht der Meßkammer von der der Membran gegenüberliegenden Seite des Gehäuses, eine Ansicht im rechten Winkel zur Membranseite, sowie eine membranseitige Ansicht,
- Fig. 3: eine als Peristaltikpumpe ausgeführte Fluidpumpe mit Rollenrad und Haltevorrichtung zur Aufnahme der Meßkammer durch seitliches Einschieben,
- Fig. 4: die in Fig. 3 dargestellte Fluidpumpe mit eingesetzter und verrasteter Meßkammer wie in Fig. 1 und 2 dargestellt,
- Fig. 5: eine detaillierte Ausführung des in Fig. 1 Gezeigten mit Führungsschienen und Einrastmitteln, wobei hier die Gehäuseform ein Parallelepiped ist und
- Fig. 6: eine detaillierte Ausführungsform des in Fig. 4 Gezeigten mit Führung des Pumpsegmentes beim Einsetzen und bei der Messkammerstabilisierung.

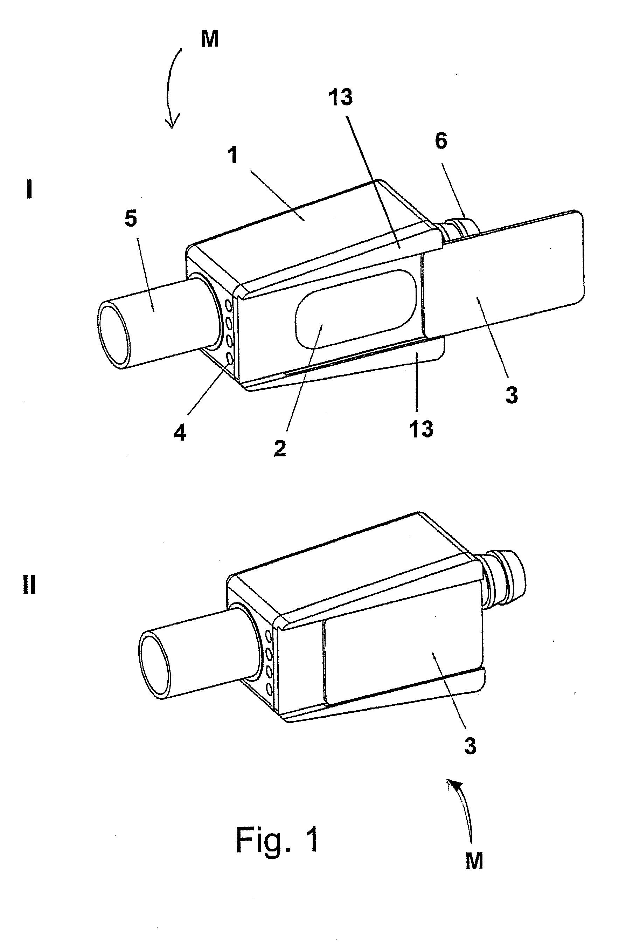

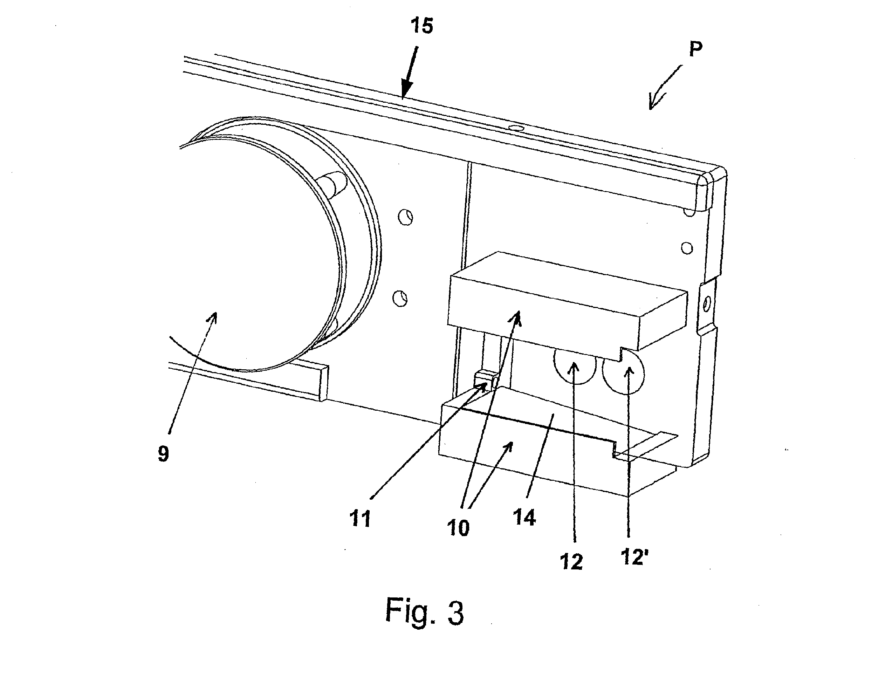

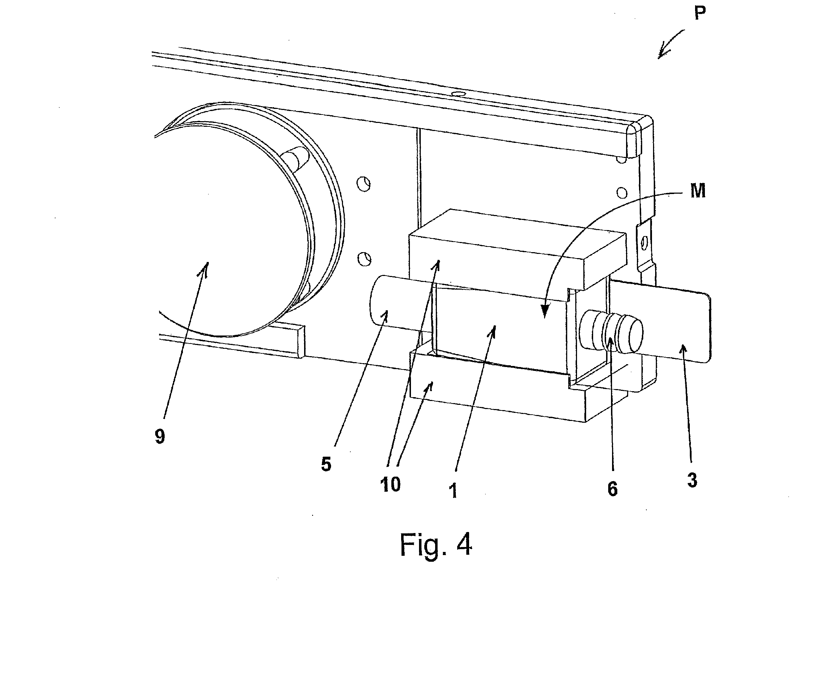

Fig. 1 zeigt ein Ausführungsbeispiel für eine erfindungsgemäße Meßkammer. Die Meßkammer weist ein blockartiges Gehäuse 1 mit ebener Oberfläche auf. An der einen Stirnseite des blockartigen Gehäuses 1 ist ein Pumpsegment 5 eingeklemmt, in die andere Stirnseite ist ein Schlauchanschluß 6 eingesetzt, die jeweils die Verlängerung eines in dieser Figur nicht näher dargestellten Strömungskanals 7 bilden. Entsprechend der Erfindung und wie später in Zusammenhang mit den Fign. 3 und 4 beschrieben wird, wird die Meßkammer bzw. das Gehäuse 1 in eine Halterung eingeschoben, die vorliegend, jedoch nicht notwendigerweise, an einer Pumpe befestigt ist.

Ebenfalls auf der das Pumpensegment 5 aufnehmenden Stirnseite des Gehäuses 1 befinden sich als Code-Stifte realisierte Codewert-Träger-Mittel 4. Diese Code-Stifte enthalten einen Codewert, der z.B. die Herstellerfirma der Meßkammer und den Querschnitt des Strömungskanales codifiziert enthält. Die Code-Stifte 4 befinden sich auf der Seite, die in Einschubrichtung der Meßkammer in die dafür vorgesehene Haltevorrichtung der Fluidpumpe liegt. In diesem speziellen Fall, nicht jedoch allgemein, ist die Einschubrichtung auch die Seite, von der das Fluid in die Meßkammer einströmt.

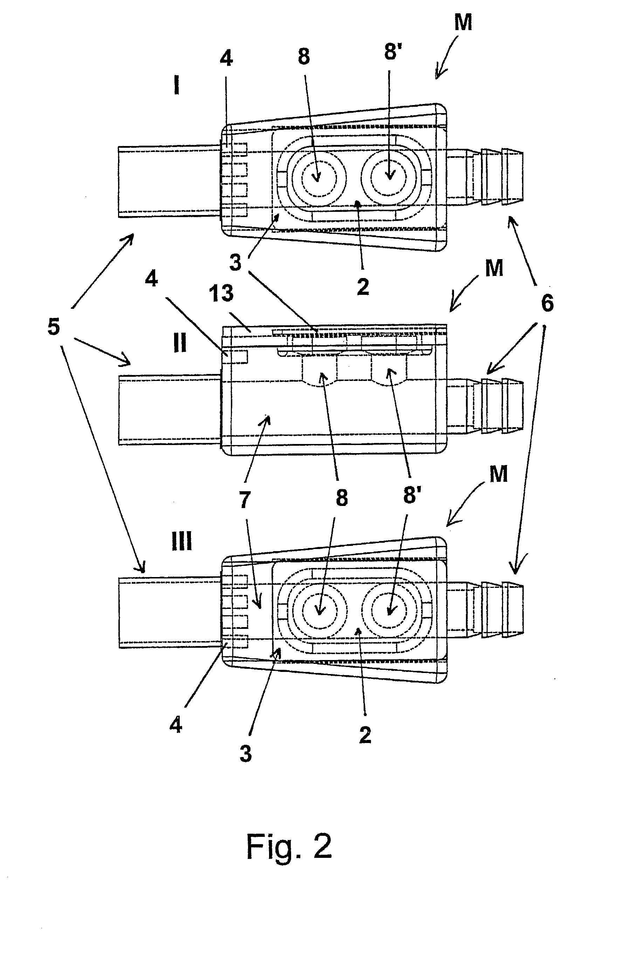

Weiterhin ist eine Membran 2 zu sehen, welche in dieser Figur nicht näher dargestellte Öffnungen 8 und 8' (siehe Fig. 2) zwischen Strömungskanal und dem Äußeren der Meßkammer abdeckt. Ein Membranschutz 3 ist hier als Platte ausgeführt, die seitlich verschiebbar ist und von aus dem Gehäuse herausspringenden Führungsschienen 13 oberhalb und unterhalb der Membranschutz-Platte 3 stabil und sicher gegen Herausrutschen geführt wird. So zeigt Fig. 1 I den Membranschutz im geöffneten Zustand und Fig. 1 II die Meßkammer mit dem Membranschutz in geschlossenem Zustand. Bei der Handhabung der Meßkammer, wenn sie nicht in die Haltevorrichtung einer Fluidpumpe eingesetzt ist, ist der Zustand wie in Fig. 1 II abgebildet der Normalzustand. Es kann hier eine Rückholkonstruktion im Gehäuse 1 vorgesehen sein, die dafür sorgt, daß der Membranschutz 3, soweit nicht eine seitliche Verschiebungskraft angewendet wird, stets in den Grundzustand wie in II abgebildet, zurückkehrt.

Fig. 2 zeigt dieselbe Meßkammer in halbtransparenter Ansicht und zwar von der der Membran gegenüber liegenden Seite her (I), in einer halbtransparenten Ansicht von senkrecht zur Membranseite (II) und von der Seite der Membran her (III).

In Fig. 2 I ist das Pumpsegment 5 zu erkennen, wie es in den Eingang des mit 7 bezeichneten Strömungskanals hineingesteckt ist. Ebenfalls deutlich sichtbar ist der Strömungsausgang in Form des Schlauchanschlusses 6. Es ist in I dargestellt, wie die Membranfläche 2, dargestellt durch die durchgezogene Linie des die Fläche 2 umgebenden abgerundeten Rechteckes, die Öffnungen 8 und 8', dargestellt als das Innere der darin liegenden gestrichelten Ringe, überdeckt. Darüber liegend ist in gestrichelter Weise der Membranschutz 3 dargestellt. Ebenfalls gestrichelt dargestellt sind die Code-Stifte 4 in der einen Stirnseite des Gehäuses.

Die zu I um 90° in der Horizontalen gedrehte Ansicht II zeigt den Membranschutz 3 in den dafür vorgesehenen Führungsschienen 13, die seitlich rechts bis nach außen fortgeführt sind, so daß der Membranschutz nach rechts über die Umgrenzung des Meßkammer-Gehäuses hinausgeschoben werden kann. Deutlich sind in dieser Ansicht auch die Öffnungen 8 und 8' zu sehen, welche die Innenseite der Membran 2 (in dieser Ansicht II nicht näher dargestellt) mit dem Strömungskanal 7 verbinden.

Die Ansicht III zeigt den geschlossenen Membranschutz 3 in durchgezogener Linie, dahinter liegend und gestrichelt die Membran 2 und die Öffnungen 8 und 8', die zum innen liegenden Strömungskanal 7 führen. Es ist ebenfalls dargestellt, wie der Membranschutz in den Führungsschienen gehalten ist. Dort wo die durchgezogene Linie der Membranschutz-Platte 3 den ebenfalls durchgezogenen oben und unten die Platte 3 umgreifenden, Vorsprung hinterfaßt, geht die durchgezogene Linie des Membranschutzes 3 in eine gestrichelte Linie über.

Eine sich zwischen den Öffnungen 8, 8' im Strömungskanal befindliche Meßdüse ist nicht näher dargestellt, ist aber besonders in der Ansicht II zwischen den Öffnungen 8 und 8' gut als eine Verengung des Strömungskanales 7 vorstellbar.

Fig. 3 zeigt ein Ausführungsbeispiel einer erfindungsgemäßen, als Peristaltikpumpe ausgeführten Fluidpumpe, welche im vorliegenden Fall darauf abgestimmt ist, die Meßkammer, wie sie im vorstehend beschriebenen Ausführungsbeispiel der Fign. 1 und 2 realisiert ist, aufzunehmen, so daß Fluidpumpe und Messkammer in erfindungsgemäßer Wirkverbindung stehen.

Andere Formen von Fluidpumpen, welche bezüglich der Wirkverbindung mit der Messkammer in analoger Weise ausgeführt sind, können einen Antrieb nach dem Peristaltikprinzip, jedoch ohne Rollenrad zur Erzeugung des Peristaltikeffekts, oder ferner einen Antrieb durch einen Rotor oder ein Flügelrad, welches seinerseits magnetisch angetrieben sein kann und auch in axialer Richtung zum Förderkanal angeordnet sein kann, enthalten. Weiterhin ist die Ausgestaltung als Zahnradpumpe mit geschlossener Zahnradkammer vorstellbar.

Im vorliegenden Ausführungsbeispiel als Peristaltikpumpe weist das Gehäuse 15 ein Rollenrad 9 auf, um das ein Schlauch herumgelegt werden kann, sowie die Haltevorrichtungen 10. Es ist damit der Förderkanal als Schlauchleitung ausgeführt.

Die Haltevorrichtungen 10 sind Ausformungen des Pumpengehäuses und so geformt, daß sie das blockartige Gehäuse 1 der Meßkammer, welches prismenförmig mit membranseitig trapezoidem Grundriß ausgeführt ist, vollständig aufnehmen und dabei sicher umfassen. Die Haltevorrichtungen 10 weisen dazu schräge Führungsflächen 14 auf, die mit den schrägen Seitenflächen des Gehäuses in Eingriff treten.

Aus in Einschubrichtung gesehenes Ende der Haltevorrichtungen 10 ist mindestens ein als Lesekontakt 11 ausgeführtes Auslesemittel angeordnet, der mit den Code-Stiften 4 zusammenarbeitet. Der mindestens eine Lesekontakt 11 und die Code-Stifte 4 können auch andere Ausführungsformen haben, nämlich in mechanischer z.B. ineinandergreifende Stifte und Löcher, elektrischer, optischer und/oder magnetischer Weise realisiert sein.

Der Lesekontakt 11 ist in der Lage, durch das mechanische Abtasten des korrespondierenden Code-Stiftes 4 der Meßkammer den darin befindlichen einstelligen Codewert auszulesen. In diesem Beispielfall wird so geprüft, ob die in die Halterung 10 einzusetzende Meßkammer mit der Pumpe kompatibel ist.

In dem Gehäuse 15 der Pumpe sind Drucksensoren 12, 12' vorgesehen, die mit der Meßkammer zusammenarbeiten. Dazu sind die Drucksensoren 12 und 12' sind so angeordnet, daß sie bei eingesetzter Meßkammer direkt in Wirkverbindung mit der vom Membranschutz 3 freigegebenen Membran 2 stehen, sobald die Meßkammer vollständig eingeschoben und verrastet ist, wobei die Drucksensoren unmittelbar im Bereich der hinter der Membran verborgenen Öffnungen 8 und 8' gegenüberliegen. Für die Verrastung der Meßkammer mit der Haltevorrichtung 10 sind nicht dargestellte Rastmittel vorgesehen, die an der Haltevorrichtung 10 und/oder dem Gehäuse 1 der Meßkammer angebracht sind.

Das Vorhandensein nur eines Auslesemittels 11, d.h. eines mechanischen Abtasters für nur einen Code-Stift, dient nur der besseren Übersichtlichkeit der Darstellung. Es können selbstverständlich auch mehrere Auslesemittel vorhanden sein, welche vorteilhaft (etwa im Einzug einer Folientastatur) übereinander in einer Reihe angeordnet sein können, wie auch die Darstellung der Code-Stifte 4 in Fig. 1 I nahelegt.

Fig. 4 zeigt die in Fig. 3 dargestellte Fluidpumpe mit eingesetzter und verrasteter Meßkammer wie in Fig. 1 I dargestellt. Zu Beginn des Einschiebevorganges wird der Membranschutz 3 von einer nicht näher dargestellten Eingriffs-Ausformung auf der Ebene der Drucksensoren 12, 12' erfaßt und festgehalten und somit die Membran 2 der Meßkammer im weiteren Verlaufe der Einschubbewegung freigegeben. In diesem vollständig eingeschobenen und verrasteten Zustand kontaktiert das Auslesemittel 11 das Codewert-Träger-Mittel 4, so daß die Steuerungsmittel des Systems, welche auch die Mittel zur Ermittlung des Fördervolumens aus den Signalen der Drucksensoren enthalten können, bestimmen, ob das System betriebsbereit ist und/oder in welcher Weise das System arbeitet. Gleichzeitig stehen die Drucksensoren 12 und 12' über der Membran 2 in Wirkverbindung mit den Öffnungen 8 und 8'.

Ist die Meßkammer mit einer geeigneten Rückholvorrichtung ausgerüstet, so schiebt sich beim Entriegeln und Entnehmen der Meßkammer aus der Haltevorrichtung der Fluidpumpe der Membranschutz 3 ohne weiteres Zutun durch den Verwender wieder schützend über die Membran. Von hier aus kann die Meßkammer ohne besondere Sorgfalt gesammelt werden und desinfiziert werden, was sich besonders im klinisch-praktischen Betrieb als vorteilhaft erweist.

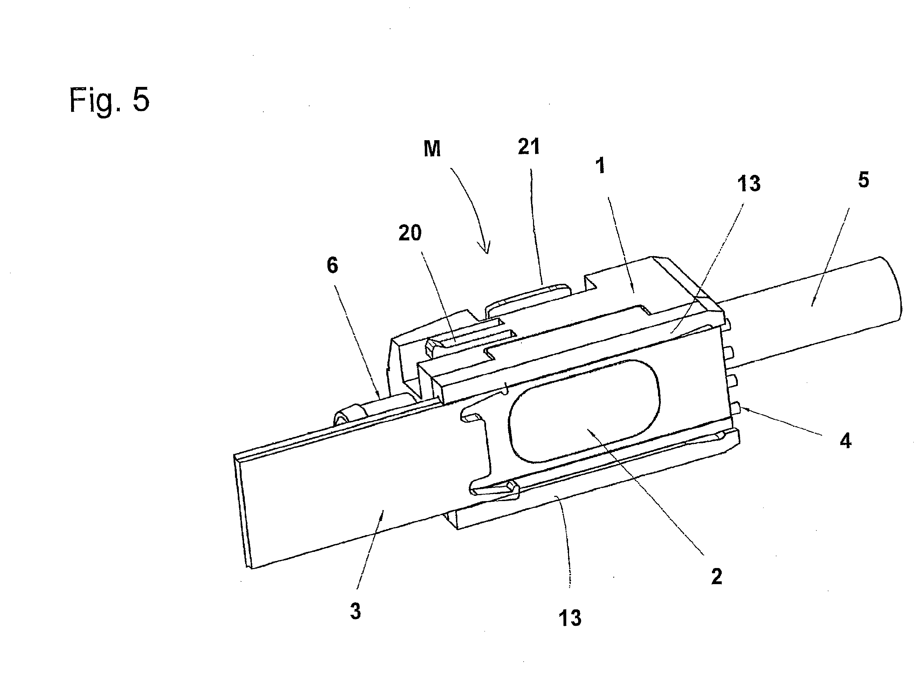

Fig. 5 zeigt eine detailliertere Ausführung des in Fig. 1 Gezeigten mit Führungsschienen 13, welche die die Membran 2 abdeckende Membranschutzplatte 3 seitlich umfassen und in denen am in der Grafik rechten Ende Ausnehmungen vorgesehen sind, in die die entsprechend ausgeformten rechten Enden der Membranschutzplatte 3 kraftschlüssig eingreifen und so die Position des Membranschutzes sichern.

Abermals dargestellt sind auch die als Code-Stifte ausgeführten Codewert-Träger-Mittel 4, die hier als konvexe, stiftartige Ausformungen des Gehäuses ausgeführt sind, sowie ferner die Einrastmittel 20, die sich jeweils an der Oberseite bzw. Unterseite des Messkammergehäuses befinden und welche beim Festlegen der Messkammer am oder im Gehäuse der Fluidpumpe mit einem deutlich vernehmbaren Klicken einrasten und die Messkammer kraftschlüssig gegen unbeabsichtigtes Lösen von der Fluidpumpe sichern. Ferner weist das Messkammergehäuse in dieser Figur eine elastische Ausformung 21 auf, welche die Messkammer nach dem als Einsetzen in eine Haltevorrichtung der Fluidpumpe realisierten Festlegen sicher innerhalb der Halterung positioniert, dadurch dass die elastischen Ausformungen 21 die in der Halterung befindliche Messkammer von der einen Seite der Halterung seitlich weg und gegen die andere Seite drücken, an der sich die Sensoren befinden, so dass keine unbeabsichtigte Bewegung des Messkammergehäuses gegenüber der Fluidpumpe stattfinden kann.

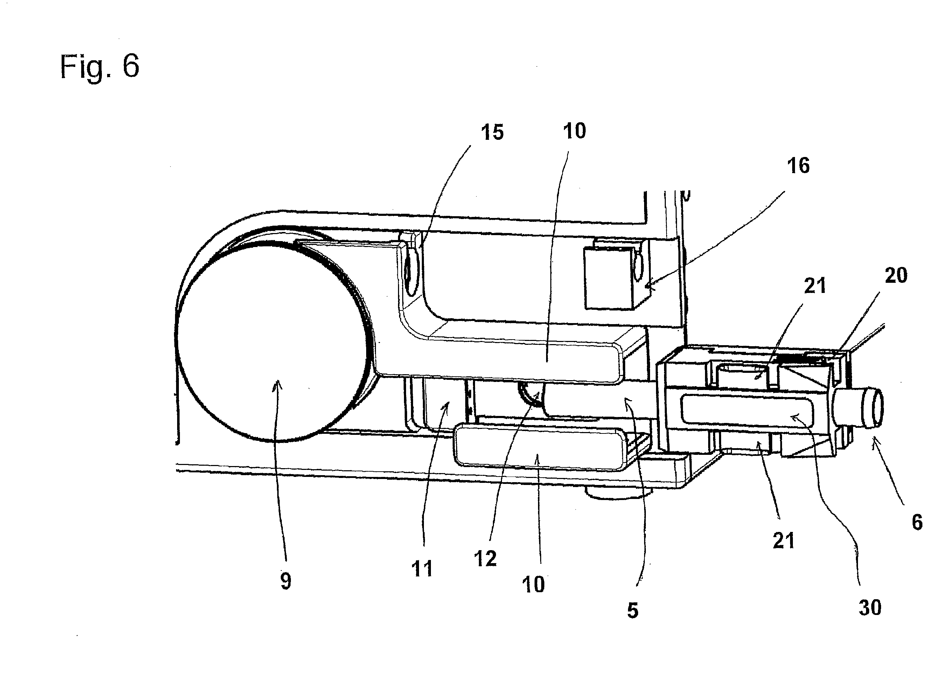

Fig. 6 zeigt, wie das Festlegen der Messkammer an einer als Peristaltikpumpe realisierten Fluidpumpe ausgeführt werden kann:

In die abgebildete Position verbracht, kann das Pumpensegment 5, welches in dieser Darstellung lediglich als kurzes Stück dargestellt ist und welches in der tatsächlichen Ausführung um ein Vielfaches länger ist, mit einer Hand ergriffen werden und gleichzeitig durch Ziehen in Richtung des Rollenrades 9 das mit dem Pumpsegment 5 verbundene Messkammergehäuse in die Haltevorrichtung 10 hineinverbracht werden. Im Zuge dieser Bewegung kann die Bedienperson des Gerätes das Pumpsegment im Uhrzeigersinn um das Rollenrad herum in die Haltevorrichtung 15 führen und schließlich in die Arretiervorrichtung 16 hineinklemmen. Somit ist das Pumpsegment stabil positioniert und die Messkammer sicher in die Haltevorrichtung verbracht. Die elastischen Ausformungen 21 sorgen hier für den Anpressdruck der Messkammer gegen die Drucksensoren 12 der Messkammer und verhindern ein unbeabsichtigtes Verrutschen, während die Einrastmittel 20 beim Einrasten ein deutlich vernehmbares Klicken erzeugen, sobald die Messkammer die betriebsbereite, an der Fluidpumpe festgelegte Position erreicht und sichert ferner das unbeabsichtigte Herausrutschen der Messkammer aus der Haltevorrichtung 11.

Als ein weiteres Merkmal der Messkammer ist in dieser Figur dargestellt, dass sie eine Etikettierfläche 30 vorsieht, auf der Information untergebracht werden kann, wie etwa das Etikett mit dem Emblem des Herstellers oder andere Informationen, z.B. der Einsatzbereich der Pumpe, etwa "nur für Uteroskopie".

## Patentansprüche

1. Fluidpumpe (P) für medizinische, insbesondere endoskopische Anwendungen, mit einem Gehäuse, einem über einen Antrieb geführten Förderkanal, einer eine Messkammer (M) aufweisenden Vorrichtung zur Messung des Drucks des in dem Förderkanal geförderten Fluids und Steuermitteln zur automatischen Steuerung der Fördereigenschaften abhängig vom gemessenen Druck, wobei die Messkammer am oder im Pumpengehäuse festlegbar ist,
**dadurch gekennzeichnet, dass**
die Messkammer (M) mit Codewert-Träger-Mitteln (4) ausgestattet ist, in welchen ein Codewert enthalten ist und dass an oder in dem Pumpengehäuse Auslesemittel (11) zur Erfassung des Codewertes während des und/oder nach dem Festlegen der Messkammer am oder im Pumpengehäuse vorgesehen sind, wobei abhängig von dem Signal der Auslesemittel und/oder dem erfassten Codewert das Verhalten der Pumpe steuerbar ist.

2. Fluidpumpe nach Anspruch 1, **dadurch gekennzeichnet, dass** die Steuermittel Funktionen zur Steuerung des Verhaltens der Pumpe abhängig vom ausgelesenen Codewert enthalten.

3. Fluidpumpe nach Anspruch 2, **dadurch gekennzeichnet, dass** das abhängig vom Codewert gesteuerte Verhalten der Pumpe die Steuerung der Fördereigenschaften der Pumpe in Abhängigkeit vom gemessenen Druck umfasst.

4. Fluidpumpe nach einem der Ansprüche 1 bis 3, **dadurch gekennzeichnet, dass** die Förderfunktion der Pumpe in Abhängigkeit vom ausgelesenen Codewert blockierbar ist.

5. Fluidpumpe nach einem der Ansprüche 1 bis 4, **dadurch gekennzeichnet, dass** die Codewert-Träger-Mittel als optische und/oder elektrische und/oder magnetische und/oder mechanische Mittel ausgebildet sind und die Auslesemittel an diese angepasst sind.

6. Fluidpumpe nach einem der Ansprüche 1 bis 5, **dadurch gekennzeichnet, dass** der in den Codewert-Träger-Mitteln enthaltene Codewert Informationen hinsichtlich der technischen Eigenschaften der Messkammer und/oder hinsichtlich des Herstellers und/oder hinsichtlich der beabsichtigen Funktionsweise der Pumpe repräsentiert.

7. Fluidpumpe nach einem der Ansprüche 1 bis 6, **dadurch gekennzeichnet, dass** die Auslesemittel elektrische Druckkontakte enthalten.

8. Fluidpumpe nach einem der Ansprüche 1 bis 7, **dadurch gekennzeichnet, dass** die Codewert-Träger-Mittel (4) den mindestens einen Codewert repräsentierende Erhebungen und/oder Vertiefungen an der Oberfläche der Messkammer enthalten.

9. Fluidpumpe nach einem der Ansprüche 1 bis 8, **dadurch gekennzeichnet, dass** die Messkammer ein Gehäuse (1) aufweist, welches Mittel zur eindeutigen Kennzeichnung der räumlichen Orientierung der Messkammer zum Pumpengehäuse aufweist.

10. Fluidpumpe nach einem der Ansprüche 1 bis 9, **dadurch gekennzeichnet, dass** die Messkammer einen Strömungskanal, welcher mit mindestens einer durch eine Membran abgedichteten Öffnung nach außen versehen ist, aufweist und dass der Öffnung gegenüberliegend mindestens ein Drucksensor in dem Pumpengehäuse angeordnet ist, der mit der Membran in Wirkverbindung steht.

11. Fluidpumpe nach Anspruch 10, **dadurch gekennzeichnet, dass** die Messkammer einen verschiebbaren Membranschutz aufweist, der die Membran abdeckt.

12. Fluidpumpe nach Anspruch 11, **dadurch gekennzeichnet, dass** der Membranschutz (3) eine die Membran mindestens im Bereich der Öffnungen bedeckende und in Führungsschienen laufende Platte (3) aufweist.

13. Fluidpumpe nach einem der Ansprüche 1 bis 12, **dadurch gekennzeichnet, dass** die Messkammer und das Pumpengehäuse Rastmittel aufweisen, die die Messkammer nach dem Festlegen am Pumpengehäuse durch Kraftschluss und/oder Formschluss halten.

14. Fluidpumpe nach einem der Ansprüche 1 bis 13, **dadurch gekennzeichnet, dass** die Messkammer einen Strömungskanal (7) aufweist und ein Pumpsegment (5) vorgesehen ist, welches eingangsseitig in den Strömungskanal (7) geklemmt ist.

15. Fluidpumpe nach einem der Ansprüche 1 bis 14, **dadurch gekennzeichnet, dass** die Messkammer einen Strömungskanal (7) aufweist und darin eine den Strömungsquerschnitt des durch den Strömungskanal (7) durchströmenden Fluids verringernde Messdüse vorgesehen ist, wobei eine Öffnung (8, 8') vor (8) und eine Öffnung nach (8') der Messdüse im Strömungskanal angeordnet sind.

16. Messkammer (M), geeignet für eine Fluidpumpe (P) nach einem der vorhergehenden Ansprüche, mit einem Gehäuse, welches einen Strömungskanal (7) einschließt,
**dadurch gekennzeichnet, dass**
sie mit Codewert-Träger-Mitteln (4) ausgestattet ist, in welchen ein Codewert enthalten ist.

17. Messkammer nach Anspruch 16, **dadurch gekennzeichnet, dass** die Codewert-Träger-Mittel als optische und/oder elektrische und/oder magnetische und/oder mechanische Mittel ausgebildet sind.

18. Messkammer nach einem der Ansprüche 16 oder 17, **dadurch gekennzeichnet, dass** die Codewert-Träger-Mittel (4) den mindestens einen Codewert repräsentierende Erhebungen und/oder Vertiefungen an der Oberfläche des Gehäuses enthalten.

19. Messkammer nach einem der Ansprüche 16 bis 18, **dadurch gekennzeichnet, dass** der in den Codewert-Träger-Mitteln enthaltene Codewert Informationen hinsichtlich der technischen Eigenschaften der Messkammer und/oder der geometrischen Abmessungen des Inneren und des Äußeren der Messkammer und/oder hinsichtlich des Herstellers und/oder hinsichtlich des beabsichtigen medizinischen Anwendungsbereiches repräsentiert.
